# EUROPEAN PATENT SPECIFICATION

(11) **EP 2 586 596 B1**
(45) Date of publication and mention of the grant of the patent: **09.08.2017**
(21) Application number: 11797981.5
(22) Date of filing: 07.06.2011
(51) Int. Cl.: B29C 67/00, B81C 1/00, A61M 37/00

(54) **THREE-DIMENSIONAL POLYMER-METAL COMPOSITE MICROSTRUCTURE AND METHOD FOR PRODUCING SAME**
DREIDIMENSIONALE POLYMER-METALL-VERBUNDMIKROSTRUKTUR UND VERFAHREN ZU IHRER HERSTELLUNG
MICROSTRUCTURE TRIDIMENSIONNELLE COMPOSITE POLYMÈRE-MÉTAL ET SON PROCÉDÉ DE PRODUCTION

(30) Priority: 24.06.2010 JP 2010143724
(43) Date of publication of application: 01.05.2013
(73) Proprietor: Japan Science and Technology Agency, Kawaguchi-shi, Saitama 332-0012 (JP)
(72) Inventor: IKUTA Koji, Nagoya-shi Aichi 462-0846 (JP); IKEUCHI Masashi, Yokohama-shi Kanagawa 222-0000 (JP)
(74) Representative: May, Mark Andrew
(86) International application number: PCT/JP2011/063024
(87) International publication number: WO 2011/162095

(56) References cited:
- JP-A- 2004 240 114
- JP-A- 2006 124 826
- JP-A- 2007 253 354
- JP-A- 2009 510 747
- JP-A- 2010 118 168
- TAKEYASU N ET AL: "Fabrication of 3D metal/polymer microstructures by site-selective metal coating", APPLIED PHYSICS A; MATERIALS SCIENCE & PROCESSING, SPRINGER, BERLIN, DE, vol. 90, no. 2, 26 October 2007 (2007-10-26), pages 205-209, XP019562022, ISSN: 1432-0630, DOI: 10.1007/S00339-007-4298-9

## Description

### [Technical Field]

The present invention relates to a three-dimensional polymer-metal microstructure and a method for producing the same. In more detail, the present invention relates to a method for producing a three-dimensional polymer-metal structure capable of being used simply and suitably for the fabrication of an operation device of cells or biomolecules, an internal drug delivery device, and the like that are a structure having a full length of from about 100 nm to 100 µm and to a three-dimensional polymer-metal micro structure formed by this method.

### BACKGROUND ART

A fine three-dimensional structure formed using a polymer, namely a three-dimensional polymer microstructure is utilized as, for example, a nanoneedle for perforating a cell membrane, other operation device of cells or biomolecules, or an internal drug delivery device. Then, in such a three-dimensional polymer microstructure, from a variety of purposes, it is attempted to subject the whole or a part of the structure to metal coating.

Hitherto, a technology for forming a device of complex fine structure between a polymer and a metal is disclosed in, for example, the following Non-Patent Document 1. Non-Patent Document 1 proposes a technique in which a metal layer is formed on a polymer layer having a photoresist, etc. formed on a substrate by means of sputtering, vapor deposition, or metal plating through a mask, and thereafter, a structure is cut out by means of chemical dissolution, focused ion beam method, or the like.

On the other hand, as techniques different from that of Non-Patent Document 1, there are those disclosed in, for example, the following Patent Documents 1 and 2 and Non-Patent Document 2. These are concerned with a technique adopting microstereolithography by means of two-photon absorption and electroless plating in combination. In the microstereolithography by means of two-photon absorption, a femtosecond pulse laser having a certain wavelength is condensed into a photocurable resin having such properties that it is cured with light having a wavelength of about a half thereof. Then, the two photon absorption is induced in only a focus center, thereby curing the polymer within a range of about 100 nm in diameter. By three-dimensionally scanning this condensing point in the photocurable resin, an arbitrary three-dimensional polymer structure can be formed.

In Patent Documents 1 and 2 and Non-Patent Document 2, a technique in which after adding an electron donor to this polymer structure in advance, or after dipping in a reducing agent after forming the polymer structure, the resulting polymer structure is dipped in an electroless plating bath, thereby depositing a metal film on the polymer structure, is further disclosed. In addition, a technique in which a photocurable resin having an electron donor added thereto and a photocurable resin not having an electron donor added thereto are used for each purpose in each site to form a complex structure, and thereafter, the polymer structure is dipped in an electroless plating bath, thereby forming a metal film in only a specified site of the polymer structure, is also disclosed.

(Patent Document 1) JP-A-2007-253354
(Patent Document 2) JP-A-2007-69406

(Non-Patent Document 1) Dan Sameoto, See-Ho Tsang, M. Parameswaran, "Polymer MEMS processing for multi-user applications", Sensors and Actuators A: Physical, 134 (2007), pp.457-464
(Non-Patent Document 2) Richard A. Farrer, Christopher N. LaFratta, Linji Li, Julie Praino, Michael J. Naughton, Bahaa E.A. Saleh, Malvin C. Teich, and John T. Fourkas, "Selective Functionalization of 3-D Polymer Microstructures", J. Am. Chem. Soc., 2006, 128, pp. 1796-1797 The publication by TAKEYASU N ET AL: "Fabrication of 3D metal/polymer microstructures by site-selective metal coating",APPLIED PHYSICS A; MATERIALS SCIENCE & PROCESSING, SPRINGER, BERLIN, DE, vol. 90, no. 2, 26 October 2007 (2007-10-26), pages 205-209, XP019562022,ISSN: 1432-0630, DOI: 10.1007/S00339-007-4298-9 is of similar content as Patent Document 1.

But, in the process disclosed in Non-Patent Document 1, a large number of steps are required, and apparatuses to be used for film formation, exposure, and cutting out are large-sized, and therefore, there was involved such a problem that the installation space and cost increase. In addition, it may be impossible to apply this process to any substrates exclusive of a planar substrate, so that there was involved such a problem that it may be impossible to form a three-dimensional structure having an arbitrary steric structure.

Next, according to the methods disclosed in Patent Documents 1 and 2 and Non-Patent Document 2, the electroless plating is adopted, and therefore, the structure must be dipped in an acid or alkali solution in a heating atmosphere. In consequence, for the purpose of fabricating an operation device of cells or biomolecules or an internal drug delivery device, in the case of mixing a biomolecule such as a nucleic acid, a protein, etc. in a photocurable resin to form a structure, there was involved such a problem that such a biomolecule is denatured. Furthermore, though it may be possible to fabricate a polymer structure having a site having a metal film formed therein and a site not having a metal film formed therein, whether or not the metal film is formed is determined depending upon the matter on whether or not the electron donor is added to the photocurable resin, and therefore, there was involved such a problem that it may be impossible to form a metal film of a different kind depending on the site of the polymer structure.

### DISCLOSURE OF THE INVENTION

A first object of the present invention is to provide a method for producing a three-dimensional polymer-metal microstructure, which is a production method capable of being easily carried out by a small number of steps at ordinary temperature and atmospheric pressure, and which is able to produce a polymer-metal structure having an arbitrary steric structure and does not denature biomolecules in a process of metal coating.

Furthermore, an object of the present invention is to provide a production method capable of forming a layer containing a metal in only an arbitrary site on a three-dimensional polymer-metal microstructure.

Furthermore, an object of the present invention is to provide a useful three-dimensional polymer-metal complex microstructure which is a resultant from such a production method.

The invention is concerned with a method for producing a three-dimensional polymer-metal microstructure comprising:
constituting a fine polymer structure having an arbitrary steric structure by stereolithography using a photocurable resin having a reactive group X; and
subsequently, dipping the polymer structure in a liquid that is an aqueous medium solution or aqueous medium dispersion liquid of a metal-containing nanoparticle having a reactive group X' capable of being bound to the reactive group X and containing an arbitrary metal, to bind the reactive group X on the polymer structure to the reactive group X' on the metal-containing nanoparticle,
thereby forming a metal-containing layer on the polymer structure.

Accordingly, it is possible to provide a method for producing a three-dimensional polymer-metal microstructure, which is a production method capable of being easily carried out by a small number of steps at ordinary temperature and atmospheric pressure, and which is able to produce a polymer-metal structure having an arbitrary steric structure and does not denature biomolecules in a process of metal coating.

A related example not forming part of the invention is concerned with a method for producing a three-dimensional polymer-metal microstructure comprising:
including a first member forming step of forming a fine first member having an arbitrary steric structure by stereolithography using a photocurable resin having a reactive group Y and a second member forming step of positioning a photocurable resin having a reactive group Z in a region including the first member and forming a fine second member having an arbitrary steric structure and connecting to the first member by stereolithography, to constitute a fine polymer structure that is an integral molded article including the first member and the second member; and
subsequently, dipping the polymer structure in (1) a first liquid that is an aqueous medium solution or aqueous medium dispersion liquid of a metal-containing nanoparticle having a reactive group Y' which is capable of being specifically bound to the reactive group Y but not capable of being bound to the reactive group Z and containing a metal A and (2) a second liquid that is an aqueous medium solution or aqueous medium dispersion liquid of a metal-containing nanoparticle having a reactive group Z' which is capable of being specifically bound to the reactive group Z but not capable of being bound to the reactive group Y and containing a metal B, respectively in random order, or dipping the polymer structure in a mixed liquid of the first liquid and the second liquid, to specifically bind the reactive group Y and the reactive group Y', and the reactive group Z and the reactive group Z', respectively to each other,
thereby forming a metal-containing layer containing the metal A in the first member of the polymer structure and a metal-containing layer containing the metal B in the second member of the polymer structure.

Accordingly, it is possible to form a layer containing a dissimilar metal in each arbitrary site on the three-dimensional polymer-metal microstructure.

Another related example not forming part of the invention is concerned with a method for producing a three-dimensional polymer-metal microstructure comprising:
including a first member forming step of forming a fine first member having an arbitrary steric structure by stereolithography using a photocurable resin having a reactive group Y and a second member forming step of positioning a photocurable resin having a reactive group Z in a region including the first member and forming a fine second member having an arbitrary steric structure and connecting to the first member by stereolithography, to constitute a fine polymer structure that is an integral molded article including the first member and the second member; and
subsequently, dipping the polymer structure in (3) a third liquid that is an aqueous medium solution or aqueous medium dispersion liquid of a metal-containing nanoparticle having a reactive group Y' which is capable of being specifically bound to the reactive group Y but not capable of being bound to the reactive group Z and containing an arbitrary metal, or (4) a fourth liquid that is an aqueous medium solution or aqueous medium dispersion liquid of a metal-containing nanoparticle having a reactive group Z' which is capable of being specifically bound to the reactive group Z but not capable of being bound to the reactive group Y and containing an arbitrary metal, to specifically bind reactive groups related to either one of a combination of the reactive group Y and the reactive group Y', and a combination of the reactive group Z and the reactive group Z',
thereby forming a metal-containing layer in only either one of the first member and the second member of the polymer structure.

Accordingly, it is possible to form a metal-containing layer in only an arbitrary site on the three-dimensional polymer-metal microstructure. Incidentally, in view of utility of a raw material body, the example brings about such an advantage that by constituting, as a raw material body, a fine polymer structure that is an integral molded article including the first member and the second member and then making selection so as to dip this raw material body in either one of the third liquid and the fourth liquid, it is possible to freely select to form the metal-containing layer on either the first member or the second member.

In an embodiment of the invention the irradiation light used for the stereolithography is an irradiation light for generating multi-photon absorption in an irradiation region in the photocurable resin.

Accordingly, it is possible to fabricate a three-dimensional polymer structure having an arbitrary shape with a curing resolution of not more than 100 nm.

In a further embodiment of the invention one or more reactive groups among the reactive groups X which the photocurable resin has and the reactive groups X' which the metal-containing nanoparticle has are protected by a protective group capable of being eliminated upon hydrolysis in the aqueous medium solution or aqueous medium dispersion liquid.

Accordingly, in the stereolithography, it is possible to prevent the wasteful consumption of the reactive groups X which the photocurable resin has, or the reactive groups X' which the metal-containing nanoparticle has, prior to the production of a three-dimensional polymer-metal microstructure.

According to another embodiment of the invention the metal contained in the metal-containing nanoparticle is one or two or more metals selected among gold, silver, and magnetic metals. Accordingly, is possible to provide a three-dimensional polymer-metal microstructure having a metal-containing layer containing one or two or more metals selected among gold, silver, and magnetic metals. For example, in the case where the three-dimensional polymer-metal microstructure is a nanoneedle for perforating a cell membrane, if a metal-containing layer of gold or silver is formed in a needle tip thereof, it can become easy and sure to perforate a cell membrane by irradiation of a laser light onto the needle tip to generate a shock wave or a cavitation bubble. Furthermore, in the case where the three-dimensional polymer-metal microstructure is a device for drug delivery movable in the body or liquid, which is constituted of at least one drug carrier part and a transportation part, if a metal-containing layer containing a magnetic metal is formed in the transportation part, it is possible to move this device easily and surely in the body or liquid due to a magnetic force from the outside.

According to yet another embodiment of the invention the constituting process of a polymer structure by stereolithography and the binding reaction of a reactive group in the aqueous medium solution or aqueous medium dispersion liquid are carried out within a temperature range of from 0 °C to 40°C and/or within a pH range of from 7 to 9.

Accordingly, since it is possible to carry out the production process of a three-dimensional polymer-metal microstructure under a very mild condition, even in the case where a biomolecule is contained in the photocurable resin, denaturation of the biomolecule can be suppressed. Incidentally, examples of the "case where a biomolecule is contained in the photocurable resin" include an example wherein the three-dimensional polymer-metal complex microstructure is constituted as a device for perforating a cell membrane to collect a target substance within the cell, and wherein the photocurable resin is allowed to contain a biomolecule such as DNA, an antigen protein, etc. in advance, and the subject biomolecule is utilized as a scavenger of the target substance; and the

The invention also concerns a three-dimensional polymer-metal microstructure comprising a polymer structure that is an integrally formed article including a first member composed of a photocurable resin and having an arbitrary steric structure and a second member composed of a photocurable resin and having an arbitrary steric structure, wherein either one of the first member and the second member has a metal-containing layer, wherein the three-dimensional polymer-metal microstructure is formed in accordance with claim 1.

Related examples which do not form part of the invention also concern a three-dimensional polymer-metal complex microstructure corresponding to any one of the following (5), (6) and (8).

(5) A fine polymer structure that is an integral molded article including a fine first member composed of a photocurable resin and having an arbitrary steric structure and a fine second member composed of a photocurable resin and having an arbitrary steric structure, wherein
a metal-containing layer containing a metal A is formed in the first member; and
a metal-containing layer containing a metal B is formed in the second member.

(6) A fine polymer structure that is an integral molded article including a fine first member composed of a photocurable resin having a reactive group Y and having an arbitrary steric structure and a fine second member composed of a photocurable resin having a reactive group Z and having an arbitrary steric structure, wherein
a metal-containing layer containing the metal A is formed in the first member through binding of a metal-containing nanoparticle having a reactive group Y' which is capable of being specifically bound to the reactive group Y and containing a metal A on the basis of binding between the reactive group Y and the reactive group Y'; and
a metal-containing layer containing the metal B is formed in the second member through binding of a metal-containing nanoparticle having a reactive group Z' which is capable of being specifically bound to the reactive group Z and containing a metal B on the basis of binding between the reactive group Z and the reactive group Z'.

(8) A fine polymer structure that is an integral molded article including a fine first member composed of a photocurable resin having a reactive group Y and having an arbitrary steric structure and a fine second member composed of a photocurable resin having a reactive group Z and having an arbitrary steric structure, wherein
a metal-containing layer containing an arbitrary metal is formed in the first member through binding of a metal-containing nanoparticle having a reactive group Y' which is capable of being specifically bound to the reactive group Y and containing the metal on the basis of binding between the reactive group Y and the reactive group Y'; or
a metal-containing layer containing an arbitrary metal is formed in the second member through binding of a metal-containing nanoparticle having a reactive group Z' which is capable of being specifically bound to the reactive group Z and containing the metal on the basis of binding between the reactive group Z and the reactive group Z'.

Accordingly, a useful three-dimensional polymer-metal complex microstructure is provided. That is, a three-dimensional polymer-metal microstructure composed of a fine polymer structure having an arbitrary shape and a metal-containing layer can be efficiently realized. According to another embodiment of the invention concerns a nanoneedle for injecting a target substance into a cell or collecting a target substance from a cell, wherein the nanoneedle is constituted of the three-dimensional polymer-metal microstructure of claim 6, wherein the metal-containing layer is formed at the needle tip of the nanoneedle.

### [Brief Description of the Drawings]

[FIG. 1] shows a process of a working example of a production method according to the present invention.
[FIG. 2] shows a specific example of protection of a reactive group by a protective group.
[FIG. 3] shows a specific example of a metal-containing nanoparticle.
[FIG. 4] is an optical microscope observation image for confirming the formation of a metal-containing layer.
[FIG. 5] is a scanning electron microscope observation image for confirming the formation of a metal-containing layer.
[FIG. 6] shows a use state of a specific example of a device using a three-dimensional polymer-metal complex microstructure.

### EXPLANATIONS OF LETTERS OR NUMERALS

- 1:: Substrate
- 2:: Photocurable resin
- 3:: First member
- 4:: Photocurable resin
- 5:: Second member
- 6:: Solution
- 7:: Metal-containing layer
- 8:: Three-dimensional polymer-metal complex microstructure
- 9:: Metal complexed nanoneedle
- 10:: Polymer structure
- 11:: Needle tip
- 12:: Metal-containing layer
- 13:: Cell
- 14:: Cell membrane

### BEST MODES FOR CARRYING OUT THE INVENTION

Next, modes for carrying out the present invention inclusive of best modes thereof are described.

### [Production method of three-dimensional polymer-metal complex microstructure]

The production method of a three-dimensional polymer-metal complex microstructure according to the present invention includes the following first production method. Each of concepts of the "photocurable resin", "stereolithography", "polymer structure", " liquid", "metal-containing nanoparticle", and "formation of metal-containing layer" related to these production methods are described later in detail.

### (First production method)

In short, a first production method of a three-dimensional polymer-metal microstructure is a method for constituting a fine polymer structure and forming a metal-containing layer on the polymer structure.

When the contents of this first production method are described more specifically, the first production method is a method for producing a three-dimensional polymer-metal complex microstructure comprising
constituting a fine polymer structure having an arbitrary steric structure by stereolithography using a photocurable resin having a reactive group X; and
subsequently, dipping the polymer structure in a liquid that is an aqueous medium solution or aqueous medium dispersion liquid of a metal-containing nanoparticle having a reactive group X' capable of being bound to the reactive group X and containing an arbitrary metal, to bind the reactive group X on the polymer structure to the reactive group X' on the metal-containing nanoparticle,
thereby forming a metal-containing layer on the polymer structure.

In the foregoing first production method, the "steric structure" which the polymer structure has is not limited so far as in short, it has a three-dimensional spatial structure. Examples thereof include, in addition to steric structures such as a spherical shape, a block shape, a rod shape, a needle shape, etc., steric structures such as a ring shape, a coil shape, a cylindrical shape, and a complicated shape in which these shape units are complexed. Furthermore, even a structure of a very thin plate shape or a sheet shape is also included so far as it substantially has a thickness. This point is also the same in the second and third production methods as described later. Incidentally, the "steric structure" of each of the first member and the second member in the second and third production methods also has the same concept.

In addition, in the first production method, the "aqueous medium" refers to a solvent composed of water, or refers to a solvent which is a mixed solvent of water and a hydrophilic solvent and in which a mixing ratio of the hydrophilic solvent is low to such an extent that a biomolecule such as a protein, etc. is not denatured. This point is also the same in the second and third production methods as described later.

In addition, in the first production method, preferably, the constituting process of a polymer structure by stereolithography and the binding reaction of a reactive group in the aqueous medium solution or aqueous medium dispersion liquid are carried out within a temperature range of from 0 °C to 40 °C and/or within a pH range of from 7 to 9. This point is also the same in the second and third production methods as described later.

### (Second production method)

In short, a second production method of a three-dimensional polymer-metal complex microstructure is a method for constituting a fine polymer structure that is an integral molded article including a first member and a second member, forming a metal-containing layer containing a metal A in the first member, and forming a metal-containing layer containing a metal B which is different from the metal A in the second member. Said second production method does not form part of the invention, however.

When the contents of this second production method are described more specifically, the second production method is a method for producing a three-dimensional polymer-metal microstructure comprising:
including a first member forming step of forming a fine first member having an arbitrary steric structure by stereolithography using a photocurable resin having a reactive group Y and a second member forming step of positioning a photocurable resin having a reactive group Z which is different from the reative group Y in a region including the first member and forming a fine second member having an arbitrary steric structure and connecting to the first member by stereolithography, to constitute a fine polymer structure that is an integral molded article including the first member and the second member; and
subsequently, dipping the polymer structure in (1) a first liquid that is an aqueous medium solution or aqueous medium dispersion liquid of a metal-containing nanoparticle having a reactive group Y' which is capable of being specifically bound to the reactive group Y but not capable of being bound to the reactive group Z and containing a metal A and (2) a second liquid that is an aqueous medium solution or aqueous medium dispersion liquid of a metal-containing nanoparticle having a reactive group Z' which is capable of being specifically bound to the reactive group Z but not capable of being bound to the reactive group Y and containing a metal B, respectively in random order, or dipping the polymer structure in a mixed liquid of the first liquid and the second liquid, to specifically bind the reactive group Y and the reactive group Y', and the reactive group Z and the reactive group Z', respectively to each other,
thereby forming a metal-containing layer containing the metal A in the first member of the polymer structure and a metal-containing layer containing the metal B in the second member of the polymer structure.

In the foregoing second production method, the terms "positioning a photocurable resin having a reactive group Z in a region including the first member" refer to (a) a form in which the already formed first member is dipped in the uncured photocurable resin having a reactive group Z, or (b) a form in which the uncured photocurable resin having a reactive group Z is positioned in a state where it comes into contact with a part of the already formed first member, or the like. This point is the same as in the third production method as described later.

In addition, in the second production method, the terms "integral molded article including the first member and the second member" refer to (a) a molded article in which the first member and the second member each having a fixed shape are directly joined with each other in a fixed positional relation, (b) a molded article in which the first member and the second member each having a fixed shape are joined with each other in a state where another member is allowed to intervene in the midway between the both, (c) a state where the first member and the second member each having a fixed shape are partially or wholly embedded in another member as a base material, without being directly joined with each other, or the like. This point is also the same as in the third production method as described later.

### (Third production method)

In short, a third production method of a three-dimensional polymer-metal complex microstructure is a method for constituting a fine polymer structure that is an integral molded article including a first member and a second member and forming a metal-containing layer in only either one of the first member and the second member. Again, the third production method does not form part of the invention.

When the contents of this third production method are described more specifically, the third production method is a method for producing a three-dimensional polymer-metal complex microstructure comprising:
including a first member forming step of forming a fine first member having an arbitrary steric structure by stereolithography using a photocurable resin having a reactive group Y and a second member forming step of positioning a photocurable resin having a reactive group Z which is different from the reactive group Y in a region including the first member and forming a fine second member having an arbitrary steric structure and connecting to the first member by stereolithography, to constitute a fine polymer structure that is an integral molded article including the first member and the second member; and subsequently, dipping the polymer structure in (3) a third liquid that is an aqueous medium solution or aqueous medium dispersion liquid of a metal-containing nanoparticle having a reactive group Y' which is capable of being specifically bound to the reactive group Y but not capable of being bound to the reactive group Z and containing an arbitrary metal, or (4) a fourth liquid that is an aqueous medium solution or aqueous medium dispersion liquid of a metal-containing nanoparticle having a reactive group Z' which is capable of being specifically bound to the reactive group Z but not capable of being bound to the reactive group Y and containing an arbitrary metal, to specifically bind reactive groups related to either one of a combination of the reactive group Y and the reactive group Y', and a combination of the reactive group Z and the reactive group Z',
thereby forming a metal-containing layer in only either one of the first member and the second member of the polymer structure.

### [Photocurable resin]

The photocurable resin refers to a resin which is a liquid in an uncured state and which when irradiated with light such as ultraviolet rays, visible light beams, etc., starts polymerization and is cured.

In general, the photocurable resin is a resin obtained by mixing a liquid monomer or oligomer with a photopolymerization initiator, a diluent, a stopping agent, a light absorber, a filler, and the like. Examples of such a monomer or oligomer include urethane acrylate based, epoxy acrylate based, acrylate based, epoxy based, vinyl ether based, and oxetane based compounds.

Though the kind of the photocurable resin which is used in the present invention is not limited, as shown in embodiments as described later, for the purpose of fabricating a fine structure, it is preferable to use a photocurable resin which is an epoxy based or oxetane based compound with less cure shrinkage and which does not contain a light scattering fine particle such as a filler, etc.

### [Stereol ithography]

The stereolithography refers to a technique for curing a liquid photocurable resin upon irradiation with light, thereby fabricating a three-dimensional structure. Though a technique for forming arbitrary shape and structure in the three-dimensional structure is not limited, in general, there is exemplified a technique in which a liquid photocurable resin is cured upon irradiation with light, and a new liquid resin is laminated on a cured portion, follower by successive curing, thereby fabricating an arbitrary three-dimensional structure. In addition, as described later, there is also exemplified a technique for omitting a lamination step by means of utilization of multi-photon absorption, or the like.

In more detail, the stereolithography is distinguished by a mode of light absorption, a scanning technique of light, and a lamination technique of photocurable resin.

As classification by the mode of light absorption, a type of absorbing one photon having a wavelength of a green to ultraviolet region to cure is general. However, for example, as disclosed in JP-A-2001-158050, there is also exemplified multi-photon absorption microstereolithography for irradiating a pulse laser of an infrared region to generate two or more multi-photon absorptions at the same time, thereby achieving curing with a minute resolution of not more than the wavelength.

As classification by the scanning technique of light, there are exemplified a technique for scanning condensed point-like light using a galvano mirror or an acoustooptic element, thereby achieving curing; and a technique for planarly making a pattern of light using a mask sheet, a liquid crystal filter, or a digital mirror device and planarly exposing it, thereby achieving curing.

The lamination technique of the photocurable resin is classified into free-surface stereolithography in which a liquid surface of the resin is exposed in the atmosphere; constrain-surface stereolithography in which a light-permeable thin plate is disposed on a liquid surface of the resin to uniformly keep the liquid surface; and a technique in which curing in a portion other than a focus is suppressed by means of utilization of multi-photon absorption, or the like, thereby omitting a lamination step.

In the present invention, though all of the foregoing techniques can be adopted, in embodiments as described later, for the purpose of efficiently fabricating an extremely fine device, the technique for utilizing multi-photon absorption as the mode of light absorption, thereby omitting a lamination step was carried out.

### [Metal-containing nanoparticle]

The metal-containing nanoparticle contains a fine metal particle having an outer diameter of, for example, from about 1 nm to 1 µm and has a reactive group which is specifically bound to a reactive group which the photocurable resin constituting the polymer structure has. In consequence, the metal-containing nanoparticle is usually composed of a metal particle and an organic molecule bound thereto (or a polymer particle having a metal particle included therein), and this organic molecule or polymer particle has a reactive group which is specifically bound to a reactive group which the photocurable resin has.

Though the kind of the metal constituting the metal particle is not limited, preferred examples thereof include gold, silver, and magnetic metals. In the foregoing second production method, a metal-containing nanoparticle containing the metal A and a metal-containing nanoparticle containing the metal B which is different from the metal A are used in combination.

### [Formation of metal-containing layer]

In the first to third production methods, the metal-containing layer in which the metal-containing nanoparticle is bound in a stratiform state is formed on the polymer structure (or on the first member and/or second member of the polymer structure) in the liquid. In consequence, the metal-containing layer is formed in a state of a surface coating layer.

It is necessary that the reactive group which the photocurable resin has and the reactive group which the metal-containing nanoparticle has are reactive groups which are specifically bound mutually. As a combination of reactive groups having such specific binding properties, those according to well-known or known combinations can be arbitrarily adopted. Examples thereof include a combination of an acidic group such as a sulfonic acid group, a carboxyl group, a thiol group, a phosphoric acid group, etc. with a basic group such as an amino group, an imino group, a maleimide group, etc. In addition, there can also be exemplified a combination of an amino group with an isothiocyanate group, a combination of an amino group with an NHS group, a combination of a thiol group with a maleimide group, a combination of biotin with avidin, a combination of biotin with streptavidin, a combination of DNA with complementary DNA, and the like.

In the second or third production method, it is required to allow the metal-containing nanoparticle for subjecting the first member to a metal coating treatment to have a reactive group Y' which is capable of being specifically bound to a reactive group Y which the photocurable resin constituting the first member has but is not capable of being bound to a reactive group Z which the photocurable resin constituting the second member has. In addition, it is required to allow the metal-containing nanoparticle for subjecting the second member to a metal coating treatment to have a reactive group Z' having reverse binding properties to the foregoing Y'.

As a combination of reactive groups satisfying such a relation, though those according to well-known or known combinations can be arbitrarily adopted, for example, the following groups can be exemplified.

The reactive group Y or Y' is selected from the group of combinations consisting of (an amino group with an isothiocyanate group), (an amino group with an NHS group), (a thiol group with a maleimide group), (biotin with avidin), (biotin with streptavidin), and (DNA with complementary DNA). Furthermore, the reactive group Z or Z' is selected from the foregoing group but other than the combination selected as the reactive group Y and Y'. However, in the case where the reactive group Y or Y' is an amino group, then an amino group cannot be included in the combination of the reactive groups Z and Z'. In addition, in the case where the reactive group Y or Y' is biotin, then biotin cannot be included in the combination of the reactive groups Z and Z'. Furthermore, in the case where the reactive groups Y and Y' are a combination of DNA with complementary DNA, and the reactive groups Z and Z' are a combination of DNA with complementary DNA, then all of the reactive group Y and the reactive group Z, and the reactive group Y' and the reactive group Z' must be a different sequence from each other.

### [Three-dimensional polymer-metal complex microstructure]

The three-dimensional polymer-metal microstructure according to the present invention is corresponding to the following (7). (5), (6) and (8) are related examples not forming part of the invention.

(5) A fine polymer structure that is an integral molded article including a fine first member composed of a photocurable resin and having an arbitrary steric structure and a fine second member composed of a photocurable resin and having an arbitrary steric structure, wherein
a metal-containing layer containing a metal A is formed in the first member; and
a metal-containing layer containing a metal B is formed in the second member.

(6) A fine polymer structure that is an integral molded article including a fine first member composed of a photocurable resin having a reactive group Y and having an arbitrary steric structure and a fine second member composed of a photocurable resin having a reactive group Z and having an arbitrary steric structure, wherein
a metal-containing layer containing the metal A is formed in the first member through binding of a metal-containing nanoparticle having a reactive group Y' which is capable of being specifically bound to the reactive group Y and containing a metal A on the basis of binding between the reactive group Y and the reactive group Y'; and
a metal-containing layer containing the metal B is formed in the second member through binding of a metal-containing nanoparticle having a reactive group Z' which is capable of being specifically bound to the reactive group Z and containing a metal B on the basis of binding between the reactive group Z and the reactive group Z'.

(7) A three-dimensional polymer-metal microstructure comprising a polymer structure that is an integrally formed article including a first member composed of a photocurable resin and having an arbitrary steric structure and a second member composed of a photocurable resin and having an arbitrary steric structure, wherein either one of the first member and the second member has a metal-containing layer, wherein the three-dimensional polymer-metal microstructure is formed in accordance with claim 1.

(8) A fine polymer structure that is an integral molded article including a fine first member composed of a photocurable resin having a reactive group Y and having an arbitrary steric structure and a fine second member composed of a photocurable resin having a reactive group Z and having an arbitrary steric structure, wherein
a metal-containing layer containing an arbitrary metal is formed in the first member through binding of a metal-containing nanoparticle having a reactive group Y' which is capable of being specifically bound to the reactive group Y and containing the metal on the basis of binding between the reactive group Y and the reactive group Y'; or
a metal-containing layer containing an arbitrary metal is formed in the second member through binding of a metal-containing nanoparticle having a reactive group Z' which is capable of being specifically bound to the reactive group Z and containing the metal on the basis of binding between the reactive group Z and the reactive group Z'.

### [Device utilizing a three-dimensional polymer-metal complex microstructure]

Though the kind of a specific device which can be utilized as the three-dimensional polymer-metal complex microstructure is not limited, as an example thereof, there can be exemplified a device in which a needle tip of a nanoneedle for perforating a cell membrane is complexed with a metal such as gold, silver, etc. by forming the foregoing metal-containing layer. In this device, it may be considered that it becomes easy and sure to perforate a cell membrane by irradiation of a laser light onto the needle tip to generate a shock wave or a cavitation bubble. In addition, as another example thereof, there is exemplified a case wherein the three-dimensional polymer-metal complex microstructure is constituted as a device for perforating a cell membrane to collect a target substance within the cell, and wherein photocurable resin is allowed to contain a biomolecule such as DNA, an antigen protein, etc. in advance, and the subject biomolecule is utilized as a scavenger of the target substance. Furthermore, as another example, there can be exemplified a case where the three-dimensional polymer-metal complex microstructure is a device for drug delivery movable in the body or liquid, which is constituted of at least one drug carrier part and a transportation part, and in which a metal-containing layer containing a magnetic metal is formed in the transportation part. This device can be moved easily and surely into, for example, a fixed target in the body or liquid due to a magnetic force from the outside.

### EXAMPLES

Example embodiment and working examples of the present invention are hereunder described. It should not be construed that the technical scope of the present invention is limited to the following Example Embodiment and Examples.

### [Example Embodiment]

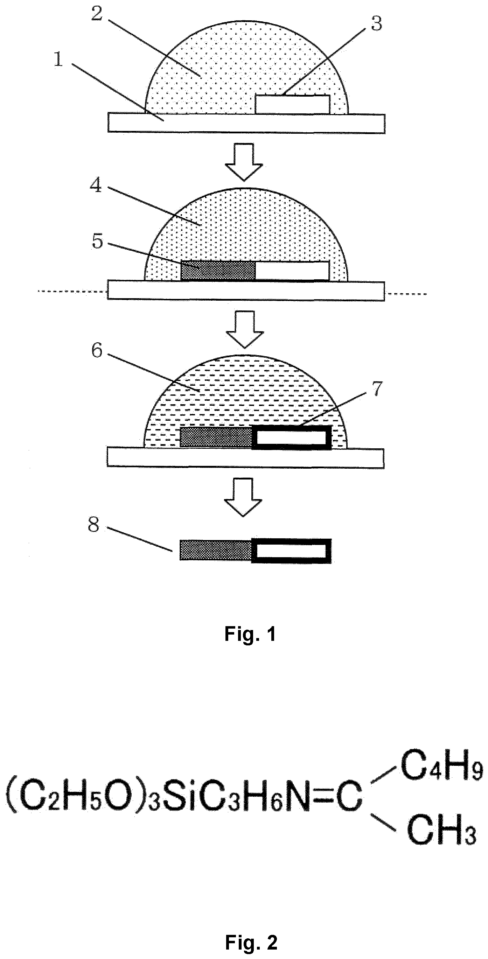

FIG. 1 shows a flowchart of an example of embodiments of the present invention.

First of all, an uncured photocurable resin 2 having a reactive group A is placed on a substrate 1, and a first member 3 of a structure is formed in the photocurable resin 2 by means of stereolithography (for example, stereolithography utilizing multi-photon absorption). After rinsing the uncured photocurable resin 2, an uncured photocurable resin 4 not having a reactive group A is placed so as to include the first member 3 therein, and a second member 5 connecting to the first member 3 is formed by means of stereolithography (for example, stereolithography utilizing multi-photon absorption), thereby obtaining a polymer structure composed of the first member 3 and the second member 5.

After rinsing the uncured photocurable resin 4, by dipping the foregoing polymer structure in a solution 6 of a metal-containing nanoparticle having an organic molecular chain having a reactive group A' which is specifically bound to the reactive group A, the reactive group A on the first member 3 is bound to the reactive group A' on the metal-containing nanoparticle, whereby a metal-containing layer 7 is formed on the first member 3. Such a metal-containing layer 7 is not formed on the second member 5. After rinsing an excess of the solution 6, the polymer structure is taken out from the substrate, thereby obtaining a three-dimensional polymer-metal complex microstructure 8.

### [Example 1]

In the present example, microstereolithography utilizing two-photon absorption and a gold nanoparticle are used.

First of all, a photocurable resin liquid (SCR710, D-MEC Ltd., JAPAN) is placed on a glass substrate (thickness: 0.15 mm), a femtosecond laser (Tsunami, Spectra-Physics, U.S.A.) having a central wavelength of 756 nm, which has been condensed with an objective lens, is irradiated in the liquid from a lower portion, and a focus is scanned by a galvano mirror, thereby forming a first site of the structure.

Subsequently, the uncured resin is removed and rinsed with diethyl ether and ethanol, a photocurable resin containing an amino group is placed on the glass substrate so as to include the foregoing first site therein, and a second site is formed by means of microstereolithography utilizing two-photon absorption.

The photocurable resin containing an amino group is a resin obtained by blending a photocurable resin (SCR710) with 15 % by weight of a silane coupling agent shown in FIG. 2 (KBE-9103, Shin-Etsu Chemical Co., Ltd., JAPAN). KBE-9103 is a silane compound in which an amino group thereof is protected. Since this protected amino group does not exhibit properties as a primary amine as it is, it is stable during the stereolithography. However, when coming into contact with water, it is extremely easily hydrolyzed to eliminate a ketone compound, thereby producing an active primary amine.

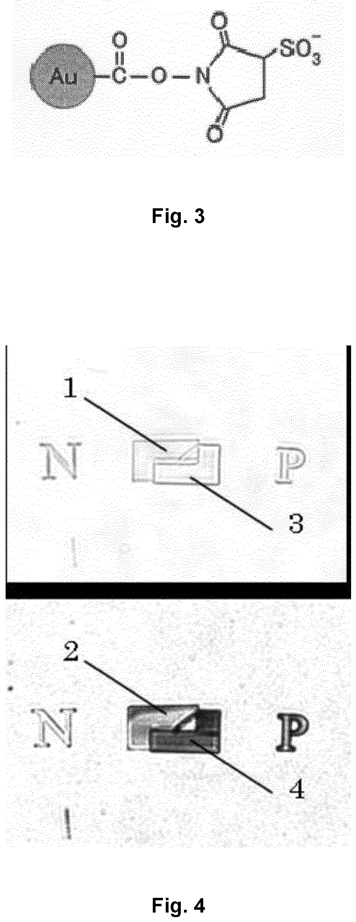

In order to form a gold-containing layer on the surface of the formed second site, after rinsing the structure with ether and ethanol, the structure is dipped in water to eliminate the protective group of the amino group, and then dipped in an aqueous solution of Mono-Sulfo-NHS-NANOGOLD (Nanoprobes) shown in FIG. 3. The present reagent is a reagent in which a sulfo-N-hydroxysuccinimide (sulfo-NHS) group is modified on a gold nanoparticle having a diameter of 1.4 nm. The sulfo-NHS group easily reacts with and binds to the amino group in an aqueous solution at a pH of from 7.5 to 8.2.

In order to confirm binding of the gold nanoparticle, a silver sensitizer was used. In a silver sensitization reaction, silver is produced with gold acting as a nucleus, and a portion where silver is produced can be confirmed as a black point upon observation with an optical microscope. FIG. 4 shows an optical observation image of the structure before and after the silver sensitization reaction. In a site not containing an amino group, a change in color between before the sensitization (1) and after the sensitization (2) is poor, whereas in a site containing an amino group, a deep black color inherent in the silver sensitization is observed after the sensitization (4) relative to before the sensitization (3), and it is exhibited that the gold particle is specifically produced in the site containing an amino group.

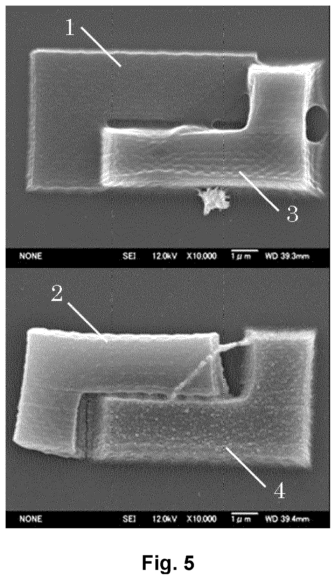

In addition, FIG. 5 shows a scanning electron microscope observation image of the surface of a structure before and after silver sensitization. In a site not containing an amino group, a change of the surface is not substantially observed between before the sensitization (1) and after the sensitization (2). On the other hand, in a site containing an amino group, a smooth surface is present before the sensitization (3), whereas the particle covers the whole of the surface after the sensitization (4). It may be considered that this is caused due to the matter that a silver particle is deposited with gold particle acting as a nucleus.

It has been demonstrated from the foregoing results that in the present embodiment, a gold-containing layer can be formed in an arbitrary site on the three-dimensional polymer microstructure fabricated by means of stereolithography in a simple and mild step.

### [Example 2]

The present example shows a use state of a metal complexed nanoneedle that is a specific example of the device using a three-dimensional polymer-metal complex microstructure.

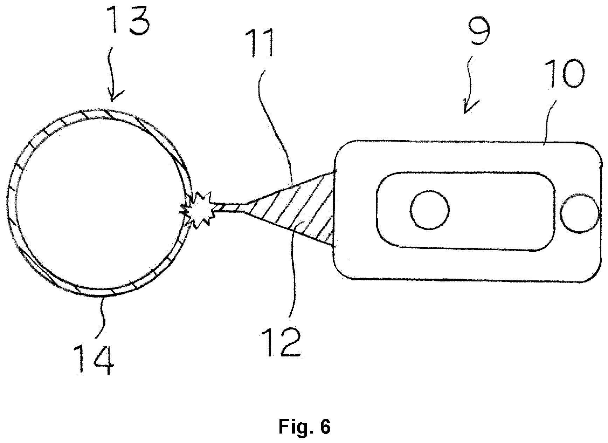

A metal complexed nanoneedle 9 that is a device shown in FIG. 6 is one in which a metal-containing layer 12 containing a metal such as gold or silver is formed in a needle tip 11 in a fine polymer structure 10 constituted in a fixed shape by means of the technique according to the present invention.

In this device, it may be considered that the injection into a cell membrane 14 of a cell 13 becomes easy and sure by irradiation of a laser light onto the needle tip 11 to generate a shock wave or a cavitation bubble (shown symbolically by a small figure such as a star shape in FIG. 6).

### INDUSTRIAL APPLICABILITY

According to the present invention, a method for producing a three-dimensional polymer-metal microstructure, which is a production method capable of being easily carried out by a small number of steps at ordinary temperature and atmospheric pressure, and which is able to produce a polymer-metal structure having an arbitrary steric structure and does not denature biomolecules in a process of metal coating is provided.

## Claims

1. A method for producing a three-dimensional polymer-metal microstructure, comprising:
constituting a polymer structure having (10) an arbitrary steric structure by stereolithography using a photocurable resin having a reactive group X; **characterized in that** the method further comprises subsequently, dipping the polymer structure (10) in a liquid that is an aqueous medium solution or aqueous medium dispersion liquid of a metal-containing nanoparticle having a reactive group X' capable of being bound to the reactive group X and containing an arbitrary metal, to bind the reactive group X on the polymer structure (10) to the reactive group X' on the metal-containing nanoparticle,
thereby forming a metal-containing layer on the polymer structure (10).

2. The method for producing a three-dimensional polymer-metal microstructure according to claim 1, wherein an irradiation light used for the stereolithography is the irradiation light for generating multi-photon absorption in an irradiation region in the photocurable resin.

3. The method for producing a three-dimensional polymer-metal microstructure according to claim 1 or 2, wherein at least one of reactive groups among the reactive group X which the photocurable resin has and the reactive group X' which the metal-containing nanoparticle has are protected by a protective group capable of being eliminated upon hydrolysis in the aqueous medium solution or aqueous medium dispersion liquid.

4. The method for producing a three-dimensional polymer-metal microstructure according to any one of claims 1 to 3, wherein the metal contained in the metal-containing nanoparticle is one or two or more metals selected among gold, silver, and magnetic metals.

5. The method for producing a three-dimensional polymer-metal microstructure according to any one of claims 1 to 4, wherein the constituting process of a polymer structure (10) by stereolithography and the binding reaction of a reactive group in the aqueous medium solution or aqueous medium dispersion liquid are carried out within a temperature range of from 0 °C to 40 °C and/or within a pH range of from 7 to 9.

6. A three-dimensional polymer-metal microstructure comprising a polymer structure (10) that is an integrally formed article including a first member composed of a photocurable resin and having an arbitrary steric structure and a second member composed of a photocurable resin and having an arbitrary steric structure,
wherein either one of the first member and the second member has a metal-containing layer (12), **characterised in that** the three-dimensional polymer-metal microstructure is formed in accordance with claim 1 .

7. A nanoneedle (9) for injecting a target substance into a cell or collecting a target substance from a cell,
wherein the nanoneedle (9) is constituted of the three-dimensional polymer-metal microstructure of claim 6,
wherein the metal-containing layer (12) is formed at the needle tip (11) of the nanoneedle (9).

8. A method for perforating a cell membrane, **characterized in that** the method comprises irradiating a laser light onto the needle tip (11) of the nanoneedle (9) of claim 7 to generate a shock wave or a cavitation bubble.

9. A drug delivery device constituted of at least one drug carrier part and a transportation part,
wherein the drug delivery device is constituted of the three-dimensional polymer-metal microstructure of claim 6, , and
wherein the transportation part comprises the metal-containing layer which contains the magnetic material.

## Patentansprüche

1. Verfahren zum Herstellen einer dreidimensionalen Polymer-Metall-Mikrostruktur, umfassend:
Bilden einer Polymerstruktur mit (10) einer beliebigen sterischen Struktur mittels Stereolithographie unter Verwendung eines photohärtbaren Harzes mit einer reaktiven Gruppe X;
**dadurch gekennzeichnet, dass** das Verfahren ferner nachfolgend ein Eintauchen der Polymerstruktur (10) in eine Flüssigkeit umfasst, welche eine Lösung eines wässrigen Mediums oder eine wässrige dispergierte Flüssigkeit eines metallhaltigen Nanopartikels mit einer reaktiven Gruppe X' ist, die in der Lage ist eine Bindung mit der reaktiven Gruppe X auszubilden und ein beliebiges Metall enthält, um die reaktive Gruppe X an der Polymerstruktur (10) an die reaktive Gruppe X' an dem metallhaltigen Nanopartikel zu binden,
wodurch eine metallhaltiger Schicht auf der Polymerstruktur (10) gebildet wird.

2. Verfahren zum Herstellen einer dreidimensionalen Polymer-Metall-Mikrostruktur nach Anspruch 1, bei dem ein bei der Stereolithographie zum Bestrahlen verwendetes Licht das Bestrahlungslicht zum Generieren einer Multiphotonenabsorption in einer Bestrahlungsregion des photohärtbaren Harzes ist.

3. Verfahren zum Herstellen einer dreidimensionalen Polymer-Metall-Mikrostruktur nach Anspruch 1 oder 2, bei dem wenigstens eine der reaktiven Gruppen der reaktiven Gruppe X, die das photohärtbare Harz aufweist, und die reaktive Gruppe X', die das metallhaltige Nanopartikel aufweist, durch eine in der Lösung des wässrigen Mediums oder der wässrigen dispergierten Flüssigkeit durch Hydrolyse eliminierbare Schutzgruppe geschützt ist.

4. Verfahren zum Herstellen einer dreidimensionalen Polymer-Metall-Mikrostruktur nach einem der Ansprüche 1 bis 3, bei dem das in dem metallhaligen Nanopartikel enthaltenen Metall ein oder zwei Metalle sind, die aus Gold, Silber und magnetischen Metallen ausgewählt ist.

5. Verfahren zum Herstellen einer dreidimensionalen Polymer-Metall-Mikrostruktur nach einem der Ansprüche 1 bis 4, bei dem der Bildungsprozess einer Polymerstruktur (10) mittels Stereolithographie und die Bindungsreaktion einer reaktiven Gruppe in der Lösung des wässrigen Mediums oder der wässrigen dispergierten Flüssigkeit in einem Temperaturbereich von 0 °C bis 40 °C und/oder in einem pH-Bereich von 7 bis 9 durchgeführt wird.

6. Dreidimensionale Polymer-Metall-Mikrostruktur, umfassend eine Polymerstruktur (10), die ein integral ausgebildeter Gegenstand ist, der ein erstes Element einschliesst, dass aus einem photohärtbaren Harz aufgebaut ist und eine beliebige sterische Struktur besitzt, und einem zweiten Element, dass aus einem photohärtbaren Harz aufgebaut ist und eine beliebige sterische Struktur besitzt,
wobei irgendeins des ersten und des zweiten Elements eine metallhaltige Schicht (12) besitzt,
**dadurch gekennzeichnet, dass** die dreidimensionale Polymer-Metall-Mikrostruktur gemäß Anspruch 1 gebildet ist.

7. Nanonadel (9) zum Injizieren einer Ziel-Substanz in eine Zelle oder zum Sammeln einer Ziel-Substanz aus einer Zelle,
wobei die Nanonadel (9) aus der dreidimensionalen Polymer-Metall-Mikrostruktur aus Anspruch 6 gebildet ist,
wobei die metallhaltige Schicht (12) an der Nadelspitze (11) der Nanonadel (9) ausgebildet ist.

8. Verfahren zum Perforieren einer Zellmembran,
**dadurch gekennzeichnet, dass** das Verfahren ein Bestrahlen der Nadelspitze (11) der Nanonadel (9) aus Anspruch 7 mit einem Laserlicht zum Erzeugen einer Schockwelle oder einer Kavitationsblase umfasst.

9. Vorrichtung zum Verabreichen von Medikamenten, gebildet aus wenigstens einem Medikamententträger-Teil und einem Transport-Teil,
wobei die Vorrichtung zum Verabreichen von Medikamenten von der dreidimensionalen Polymer-Metall-Mikrostruktur aus Anspruch 6 gebildet ist, und
wobei der Transport-Teil die metallhaltige Schicht umfasst, die das magnetische Metall enthält.

## Revendications

1. Procédé de production d'une microstructure de polymère-métal en trois dimensions, comprenant :
la réalisation d'une structure de polymère (10) ayant une structure stérique arbitraire par stéréolithographie en utilisant une résine photodurcissable ayant un groupe réactif X, **caractérisé en ce que** le procédé comprend en outre
par la suite, l'immersion de la structure de polymère (10) dans un liquide qui est une solution en milieu aqueux ou un liquide de dispersion en milieu aqueux d'une nanoparticule contenant un métal ayant un groupe réactif X' susceptible de se lier au groupe réactif X et contenant un métal arbitraire, pour lier le groupe réactif X sur la structure polymère (10) au groupe réactif X' sur la nanoparticule contenant du métal,
formant ainsi une couche contenant du métal sur la structure de polymère (10).

2. Procédé de production d'une microstructure de polymère-métal en trois dimensions selon la revendication 1, dans lequel une lumière d'irradiation utilisée pour la stéréolithographie est la lumière d'irradiation utilisée pour générer une absorption multi-photons dans une région d'irradiation de la résine photodurcissable.

3. Procédé de production d'une microstructure de polymère-métal en trois dimensions selon la revendication 1 ou 2, dans lequel au moins un des groupes réactifs parmi le groupe réactif X que comprend la résine photodurcissable et le groupe réactif X' que comprend la nanoparticule contenant du métal est protégé par un groupe protecteur susceptible d'être éliminé lors de l'hydrolyse de la solution en milieu aqueux ou le liquide de dispersion en milieu aqueux.

4. Procédé de production d'une microstructure de polymère-métal en trois dimensions selon l'une quelconque des revendications 1 à 3, dans lequel le métal contenu dans la nanoparticule contenant du métal est un ou deux métaux ou plus choisis parmi l'or, l'argent et les métaux magnétiques.

5. Procédé de production d'une microstructure de polymère-métal en trois dimensions selon l'une quelconque des revendications 1 à 4, dans lequel le procédé de réalisation d'une structure de polymère (10) par stéréolithographie et la réaction de liaison d'un groupe réactif de la solution en milieu aqueux ou le liquide de dispersion en milieu aqueux sont réalisés dans une plage de température de 0 °C à 40°C et/ou dans une plage de pH de 7 à 9.

6. Microstructure de polymère-métal en trois dimensions comprenant une structure de polymère (10) qui est un article intégralement formé comprenant un premier élément se composant d'une résine photodurcissable et ayant une structure stérique arbitraire et d'un second élément se composant d'une résine photodurcissable et ayant une structure stérique arbitraire,
dans laquelle le premier élément ou le second élément comprend une couche contenant du métal (12), **caractérisée en ce que** la microstructure de polymère-métal en trois dimensions est formée selon la revendication 1.

7. Nano-aiguille (9) pour l'injection d'une substance cible dans une cellule ou le prélèvement d'une substance cible dans une cellule,
dans laquelle la nano-aiguille (9) se compose de la microstructure de polymère-métal en trois dimensions selon la revendication 6,
dans laquelle la couche contenant du métal (12) est formée au niveau de la pointe d'aiguille (11) de la nano-aiguille (9).

8. Procédé de perforation d'une membrane cellulaire,
**caractérisé en ce que**
le procédé comprend l'irradiation d'une lumière laser sur la pointe d'aiguille (11) de la nano-aiguille (9) selon la revendication 7 pour générer une onde de choc ou une bulle de cavitation.

9. Dispositif d'administration de médicament se composant d'au moins une partie comportant le médicament et d'une partie de transport,
dans lequel le dispositif d'administration de médicament se compose de la microstructure de polymère-métal en trois dimensions selon la revendication 6, et
dans lequel la partie de transport comprend la couche contenant du métal qui contient le matériau magnétique.
